# EUROPEAN PATENT SPECIFICATION

(11) **EP 1 689 885 B1**
(45) Date of publication and mention of the grant of the patent: **17.10.2012**
(21) Application number: 04789594.1
(22) Date of filing: 20.10.2004
(51) Int. Cl.: C12Q 1/68

(54) **Assessment of predisposition to cancer by quantitative determination of methylation in normal cells of healthy persons.**
Beurteilung der Veranlagung eine Krebskrankheit zu bekommen mittels quantitativer Bestimmung der Methylierung in normalen Zellen von gesunden Menschen
Evaluation de la prédisposition de cancer par la détermination quantitative de la méthylation dans des cellules normales d'hommes sains

(30) Priority: 20.10.2003 US 513097 P; 20.10.2003 AU 2003905747
(43) Date of publication of application: 16.08.2006
(73) Proprietor: ST VINCENT'S HOSPITAL SYDNEY LIMITED, Darlinghurst, NSW 2010 (AU); Victor Chang Cardiac Research Institute, Darlinghurst, NSW 2010 (AU)
(72) Inventor: WARD, Robyn, Lynne, Woollahra, NSW 2025 (AU); MARTIN, David, I., K., Paddington, NSW 2021 (AU); SUTER, Catherine, Mary, Enmore, NSW 2042 (AU)
(74) Representative: Plougmann & Vingtoft A/S
(86) International application number: PCT/AU2004/001434
(87) International publication number: WO 2005/038047

(56) References cited:
- WO-A-00/26401
- WO-A-01/75172
- WO-A-2005/001141
- WO-A2-01/81622
- US-A1- 2003 124 600
- US-B1- 6 235 474
- CUI H ET AL: "Loss of imprinting in colorectal cancer linked to hypomethylation of H19 and IGF2" CANCER RESEARCH, AMERICAN ASSOCIATION FOR CANCER RESEARCH, BALTIMORE, MD, US, vol. 62, 15 November 2002 (2002-11-15), pages 6442-6446, XP002953235 ISSN: 0008-5472
- SUTER CATHERINE M ET AL: "HYPOMETHYLATION OF L1 RETROTRANSPOSONS IN COLORECTAL CANCER AND ADJACENT NORMAL TISSUE" INTERNATIONAL JOURNAL OF COLORECTAL DISEASE, SPRINGER VERLAG, BERLIN, DE, vol. 19, no. 2, March 2004 (2004-03), pages 95-101, XP009080008 ISSN: 0179-1958
- GRADY W M ET AL: "Detection of aberrantly methylated hMLH1 promoter DNA in the serum of patients with microsatellite unstable colon cancer." CANCER RESEARCH 1 FEB 2001, vol. 61, no. 3, 1 February 2001 (2001-02-01), pages 900-902, XP002426336 ISSN: 0008-5472
- SEEDHOUSE CLAIRE H ET AL: "Detailed methylation analysis of the mismatch repair genes hMLH1 and hMSH2 in acute myeloblastic leukemia" BLOOD, vol. 96, no. 11 Part 1, 16 November 2000 (2000-11-16), page 114a, XP009081140 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- SUTER CATHERINE M ET AL: "Germline epimutation of MLH1 in individuals with multiple cancers" NATURE GENETICS, vol. 36, no. 5, May 2004 (2004-05), pages 497-501, XP002426227 ISSN: 1061-4036
- MIYAKURA ET AL: "Extensive but hemiallelic methylation of the hMLH1 promoter region in early-onset sporadic colon cancers with microsatellite instability" CLINICAL GASTROENTEROLOGY AND HEPATOLOGY, AMERICAN GASTROENTEROLOGICAL ASSOCIATION,, US, vol. 2, no. 2, February 2004 (2004-02), pages 147-156, XP005120483 ISSN: 1542-3565
- GAZZOLI ISABELLA ET AL: "A hereditary nonpolyposis colorectal carcinoma case associated with hypermethylation of the MLH1 gene in normal tissue and loss of heterozygosity of the unmethylated allele in the resulting microsatellite instability-high tumor" CANCER RESEARCH, vol. 62, no. 14, 15 July 2002 (2002-07-15), pages 3925-3928, XP002437844 ISSN: 0008-5472
- ESTELLER M ET AL: "Analysis of adenomatous polyposis coli promoter hypermethylation in human cancer" CANCER RESEARCH, AMERICAN ASSOCIATION FOR CANCER RESEARCH, BALTIMORE, MD, US, vol. 60, no. 16, 15 August 2000 (2000-08-15), pages 4366-4371, XP002305018 ISSN: 0008-5472
- BEGUM REHANA ET AL: "Folate status and risk of colorectal polyps in African Americans" GASTROENTEROLOGY, vol. 126, no. 4, Suppl. 2, April 2004 (2004-04), page A658, XP009085256 & DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085
- HARDEN SUSAN V ET AL: "Molecular assessment of histologically negative lymph nodes and correlation with survival in surgically resected stage I lung cancer using promoter methylation of multiple genes" PROCEEDINGS OF THE AMERICAN ASSOCIATION FOR CANCER RESEARCH ANNUAL MEETING, vol. 43, March 2002 (2002-03), page 221, XP001536455 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- WIDSCHWENDTER MARTIN ET AL: "The potential prognostic, predictive, and therapeutic values of DNA methylation in cancer. Commentary re: J. Kwong et al., Promoter hypermethylation of multiple genes in nasopharyngeal carcinoma. Clin. Cancer Res., 8: 131-137, 2002, and H-Z. Zou et al., Detection of aberrant p16 methylation in the s" CLINICAL CANCER RESEARCH : AN OFFICIAL JOURNAL OF THE AMERICAN ASSOCIATION FOR CANCER RESEARCH JAN 2002, vol. 8, no. 1, January 2002 (2002-01), pages 17-21, XP002437845 ISSN: 1078-0432
- WONG I H N ET AL: "Quantitative analysis of tumor-derived methylated p16INK4a sequences in plasma, serum, and blood cells of hepatocellular carcinoma patients" CLINICAL CANCER RESEARCH, THE AMERICAN ASSOCIATION FOR CANCER RESEARCH, US, vol. 9, no. 3, March 2003 (2003-03), pages 1047-1052, XP002395878 ISSN: 1078-0432
- HIBI K ET AL: "A cancer-prone case with a background of methylation of p16 tumor suppressor gene" CLINICAL CANCER RESEARCH 01 MAR 2003 UNITED STATES, vol. 9, no. 3, 1 March 2003 (2003-03-01), pages 1053-1056, XP002437846 ISSN: 1078-0432
- ISSA J P ET AL: "Accelerated age-related CpG island methylation in ulcerative colitis." CANCER RESEARCH 1 MAY 2001, vol. 61, no. 9, 1 May 2001 (2001-05-01), pages 3573-3577, XP002437847 ISSN: 0008-5472
- HOLST C.R. T AL.: 'Methylation of p16INK4 a promoters occurs in vivo in histologically normal human mammary epithelia' CANCER RESEARCH vol. 63, no. 7, April 2003, pages 1596 - 1601, XP003016345
- NAKAYAMA H. ET AL.: 'Molecular detection of p16 promoter methylation in the serum of colorectal cancer patients' CANCER LETTERS vol. 188, no. 1-2, 15 December 2002, pages 115 - 119, XP003016346
- KUISMANEN S.A. ET AL.: 'Genetic and epigenetic modification of MLH1 accounts for a major share of microsatelite-unstable colorectal cancers' AMERICAN JOURNAL OF PATHOLOGY vol. 156, no. 5, 2000, pages 1773 - 1779, XP003016347
- J. M. Allan: "Polymorphism in glutathione S-transferase P1 is associated with susceptibility to chemotherapy-induced leukemia", Proceedings of the National Academy of Sciences, vol. 98, no. 20, 25 September 2001 (2001-09-25), pages 11592-11597, XP55021877, ISSN: 0027-8424, DOI: 10.1073/pnas.191211198

## Description

### FIELD OF THE INVENTION

The present invention relates to an assay for assessing the risk of disease (eg cancer) in an individual. In particular, the present invention relates to an assay for assessing the risk of disease comprising quantitatively determining the frequency of an epimutation in a particular gene in a population of cells from normal tissue of an individual, wherein epimutation of the gene is associated with one or more diseases.

### BACKGROUND OF THE INVENTION

### Epigenetic modifications and gene expression

Epigenetic modifications are molecular events that result in alterations in gene function that are mediated by factors other than a change in DNA sequence. Epigenetic effects on gene function commonly result in transcriptional silencing of the gene that may be maintained through mitosis, producing clonal patterns of transcriptional silence. Silencing may occur with a probability that is somewhere between 0 and 1, producing, in a single multicellular organism, a mosaic pattern of gene expression (or silence). This mosaic expression occurs despite all cells having the same genetic makeup. In some cases, epigenetic modifications are maintained in the germ-line, producing heritable effects ("epigenetic inheritance"). The molecular basis of epigenetic effects is much more complex than the simple 4-base code in DNA, and for this reason, epigenetic inheritance occurs in patterns that are much different from the simple patterns of Mendelian inheritance.

One of the best known epigenetic modifications is cytosine methylation ("DNA methylation"), which is indispensable for normal human development and is involved in the normal physiological processes of parental imprinting, suppression of transposable elements, and X-inactivation in females (reviewed in Jones and Takai 2001, and Bird 2002). In all mammals, cytosine methylation occurs essentially within the dinucleotide CpG. In the human genome, the majority of cytosine residues within CpG dinucleotides are methylated, but small proportions are maintained as unmethylated in certain CpG-rich regions called "CpG islands" (Antequera and Bird 1993). CpG islands are frequently associated with the regulatory regions of cellular genes, and a large proportion of human genes include a CpG island at their 5' end.

Histone and chromatin structure changes are other epigenetic modifications which affect gene expression. Indeed, both of these kinds of epigenetic modifications have been found to have a great impact on gene expression that is linked, although not exclusive to, DNA methylation within CpG islands (Jenuwein and Allis 2001). For example, transcriptionally active genes are generally associated with the acetylation of the fourth lysine (K⁴) of the histone subunit 3 (H3K⁴), whereas silent and methylated genes are correlated with de-acetylated H3K⁴, methylation of H3K⁹, and recruitment of the HP1 chromodomain (Kouzarides 2002). In fact, recent evidence indicates that DNA methylation occurs in response to a change in chromatin structure that is largely dictated by modifications to these key histone subunits (Tamaru and Selker 2001), and indicates that the role of DNA methylation is a consolidation of the already silent state. Therefore, DNA methylation can be regarded as a "signature" of a stably silenced genetic locus.

DNA methylation is not, however, an absolute requirement or "signature" for gene silencing since many non-human species which are devoid of CpG methylation still exhibit epigenetic silencing phenomena. Therefore, in the human genome, there are presumably many genes devoid of CpG island promoters that will still be susceptible to epigenetic modification mediated by changes in histones and chromatin structure, rather than DNA methylation. The ease and low cost with which DNA methylation can be assayed, however, makes it an attractive target to search for epigenetic modifications in humans.

### Methods to detect epigenetic changes

As described above, the simplest way to determine an epigenetic change is to test for CpG methylation. Traditionally, CpG methylation analysis has been carried out by Southern hybridisation, which assesses methylation-sensitive restriction enzyme sites within CpG islands of known genes, however, recently, more sophisticated methods for determining CpG methylation such as COBRA (combined bisulfite restriction analysis; Xiong and Laird 1997), bisulfite allelic sequencing (Frommer et al 1992), and MSP (methylation-specific PCR), have become available and allowed a more detailed analysis of CpG methylation across a CpG island of interest.

In particular, bisulfite modification of DNA now allows the discrimination of methylated CpG from unmethylated CpG, since the bisulfite treatment converts unmethylated cytosine to uracil through deamination whereas 5-methylcytosine is protected from deamination and thereby remains unchanged. Following treatment with bisulfite, the method requires that the bisulfite-modified sequence be amplified by PCR with strand-specific primers to yield a product in which uracil residues are amplified as thymine, and only 5-methylcytosine residues are amplified as cytosine. The PCR products can then be readily digested with restriction enzymes to distinguish methylated from unmethylated alleles (COBRA), or cloned and sequenced to provide methylation maps of individual DNA strands.

MSP, another widely used methylation assay method, can assess the methylation status of CpG sites within a CpG island, independent of the use of methylation-sensitive restriction enzymes. In this method, bisulfite modification is followed by amplification with primers specific for methylated DNA only, and results in the amplification of any hypermethylated alleles within a given sequence (US Patent No 5,786,146).

Further, methods for detecting epigenetic modifications are not limited to analysis of CpG methylation. That is, epigenetic modifications can also be detected by various methods which assay specific proteins bound to transcriptionally active or silent regions of DNA, or protein modifications associated with active or silent states (eg detection of specific modifications of histones, and the detection of other proteins such as homologues of HP1). At present, these protein modifications are generally assayed by immunoprecipitation with antibodies and subsequent analysis for DNA sequences present in the precipitated material.

### Genetic basis of disease

Efforts to predict predisposition for a disease, such as cancer, have been to date based largely on age, personal or family history, or occasionally by the inheritance of genetic abnormalities (ie germ-line mutations). For example, mutations in the BRCA genes are present in around half of all individuals with a strong family history of breast cancers. However, this accounts for less than 1% of breast cancers overall, and in the great majority of remaining cases there is no familial pattern consistent with a defect in a single gene. A similar situation exists with a large number of other diseases. Indeed, Table 1 provides a list of over sixty diseases which have been linked to certain genes, but where only a relatively small proportion of cases can be explained by a single predisposing genetic change.

A common explanation for diseases that have some familial pattern indicating inheritance, but no evidence for a single predisposing genetic change, is that disease results from the interaction of multiple genes. In line with this, a trait (which can manifest as disease) may be produced by the combined action of several genes, but only certain alleles of those genes will contribute to the trait. Such traits have been termed "quantitative traits", "complex traits" and "polygenic traits", and diseases that may result from such a mechanism are typically known as "multifactorial" or "polygenic" (for reviews, see Risch 2000, and Botstein and Risch 2003).

Genetic studies can not therefore, predict predisposition to disease in most cases. However, the fact that only a few patients in a family showing a strong history of a disease such as breast cancer are likely to show a single predisposing genetic change, suggests that many individuals have an innate predisposition to cancer, the basis of which is currently unknown.

### Epimutations

A gene may be inactivated by epigenetic modification. The term "epimutation" was first defined by Holliday (Holliday 1987) as a "mitotically heritable change in the methylation of a gene", however the term has since been extended to refer also to the other types of epigenetic modifications. As used herein, the term "epimutation" refers to any abnormal silencing of gene expression, in the absence of DNA sequence alteration. This definition specifically excludes abnormal silencing of a gene that is normally subject to parental imprinting (also termed "genomic imprinting" or simply "imprinting"). Parental imprinting is a normal process that involves changes in the transcription state of one allele of a gene determined by the parental origin of the allele (ie a change in the transcription state of one allele of a gene that is normally subject to parent of origin-specific expression). This process sometimes is aberrant, resulting in the loss of monoallelic expression and thus expression that is either biallelic or completely absent.

### Prior art

To date, the only clear case of a germ-line epimutation comes from a naturally occurring variant of the flowering plant, toadflax (*Linaria vulgaris*) (Cubas et al 1999). In this example, biallelic methylation and transcriptional silencing of a gene controlling symmetry, *Lcyc*, was found to be the cause of an alteration in flower phenotype. The phenotype was found to be somewhat unstable with a tendency to revert (ie some flowers on mutant plants exhibited a wild type appearance and this was associated with a loss of *Lcyc*methylation). Plants exhibiting the mutant phenotype were able to transmit the epimutation to their progeny through the germ-line. Germ-line and soma are not well separated in plants however, and this may explain the relatively stable existence of this epimutation over at least 250 years.

Epimutations are common in tumour cells. There is now a large body of literature documenting epimutations in many types of tumour, and their inverse relationship to activity of the affected gene (for reviews, see Jones and Laird 1999, Wolffe and Matzke 1999, and Baylin and Herman 2000). In some cases, the epigenetic silencing of tumour suppressor genes gives rise to distinct tumour phenotypes. For example, in sporadic colorectal cancer, around 15% of tumours exhibit microsatellite instability (MSI). MSI is a hallmark of defective mismatch repair, but only a tiny fraction of these cancers will be explained by a genetic alteration in a mismatch repair gene. It is now known that bi-allelic methylation of the *hMLH1* gene promoter is responsible for MSI tumours in the majority of cases (Herman et al 1998, and Wheeler et al 1999). MSI colorectal cancers also exhibit loss of imprinting (LOI) at IGF2 (Cui et al 1998), which may also have an epigenetic basis (Cui et al 2002). It has also been demonstrated that LOI could be detected not only in MSI tumours, but also in the normal tissues of such patients, including their peripheral blood (Cui et al 2003). The finding of LOI in peripheral blood in a small number of normal controls, and a large percentage of colorectal cancer patients has led to the hypothesis that LOI in peripheral blood is an indicator of colorectal cancer risk (US Patent No 6,235,474).

Germ-line epimutations have not yet been described in humans, although a related phenomenon can be observed in a particular strain of inbred mice, the agouti viable yellow (A^{vy}). These mice carry the A^{vy} allele, in which an intracisternal A particle (IAP) retrotransposon is inserted at the 5' end of the agouti (A) gene (Duhl et al 1994). When the IAP is epigenetically active, agouti transcription is initiated from a cryptic promoter within the 5'LTR of the IAP. The tight tissue-specific expression of agouti is abrogated by the IAP, whose LTR is active in many or all tissues and, as a result, agouti may be expressed pancellularly in A^{vy} mice. It has been found that the CpG methylation of this IAP is inversely correlated with ectopic agouti expression, and this epigenetic modification appears to gives rise to a variation in phenotype in A^{vy} mice which includes not only yellow coat colour, but also obesity, Type II diabetes, and tumour susceptibility. Significantly, this phenotype is mosaic in many individuals, indicating that the IAP is active in some cells, and silent in others, in a clonal pattern. Further, recent data indicates that there is incomplete erasure of the A^{vy} IAP methylation between generations resulting in partial maternal inheritance of the epigenotype (Morgan et al 1999). This suggests that germ-line transmission of an epigenetic modification is possible in mammals.

### Need for methods for assessing disease risk

New and improved methods for assessing disease risk in individuals (ie predicting predisposition for a disease) are desirable. That is, knowledge of disease risk may allow for the adoption of preventative therapies and avoidance of disease risk factors, and may further assist in the identification of preferred therapies upon commencement of the disease or symptoms. Preferably, methods for assessing risk are relatively simple and either non-invasive or cause only minimal discomfort to individuals.

The present applicants have detected epimutation (ie CpG methylation) in the promoter of the tumour suppressor gene *hMLH1,* in normal tissues (eg peripheral blood) of cancer patients with tumours showing a loss of the hMLH1 protein, and have found, surprisingly, that the frequency of the detected epimutation in the cells of such normal tissues is predictive of the level of cancer risk.

### SUMMARY OF THE INVENTION

Thus, the present invention provides an assay for assessing the predisposition to a cancer in a healthy individual as defined in the claims

### BRIEF DESCRIPTION OF THE FIGURES

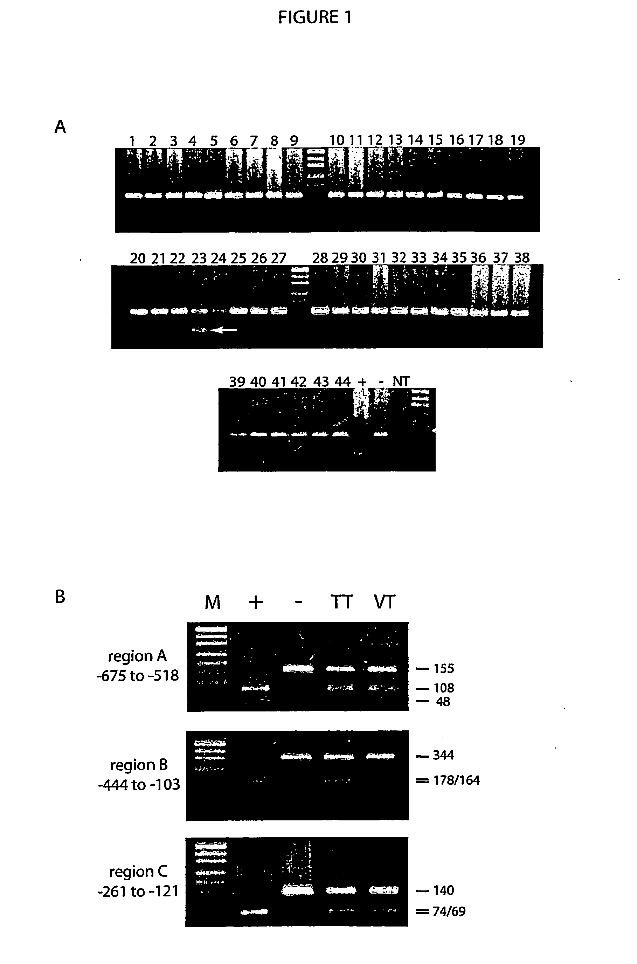

**Figure 1** shows *hMLH1* COBRA methylation analysis in peripheral blood in Example 1. (A) This photograph shows an example of the COBRA screening assay. In particular, results are shown from the C region COBRA in peripheral blood DNA from 44 cancer patients. In this subset, one patient showed methylation of *hMLH1* in the C region demonstrated by digestion of the PCR product (upper band) to yield two smaller fragments, which appear as one band (arrow). (B) These photographs show the A, B and C region COBRA results for the peripheral blood of individuals VT and TT. The location of each region relative to the transcription start site is shown on the left. For each region, digestion of the PCR product (upper band) to yield smaller fragments is indicative of methylation within that region. +, RKO cell line; -, healthy control blood DNA.

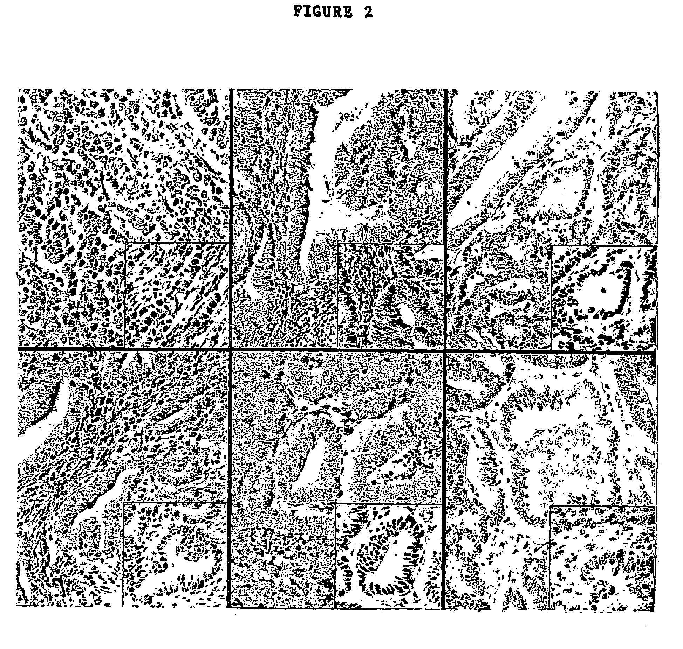

**Figure 2** shows immunohistochemical analysis of hMLH1 expression in representative cancers from the two individuals in Example 1. Carcinomas in the upper panel are from VT (left = breast, middle = endometrium, right = colon) and those in the lower panel are from TT (left = colon, middle = ampulla of Vater. Right = duodenal). All cancers showed complete loss of hMLH1 expression. For all tumours, the inset shows positive staining of the same tumour for hMSH2. Immunoperoxidase with haematoxylin counterstain; Bar (lower left) represents 100 µm.

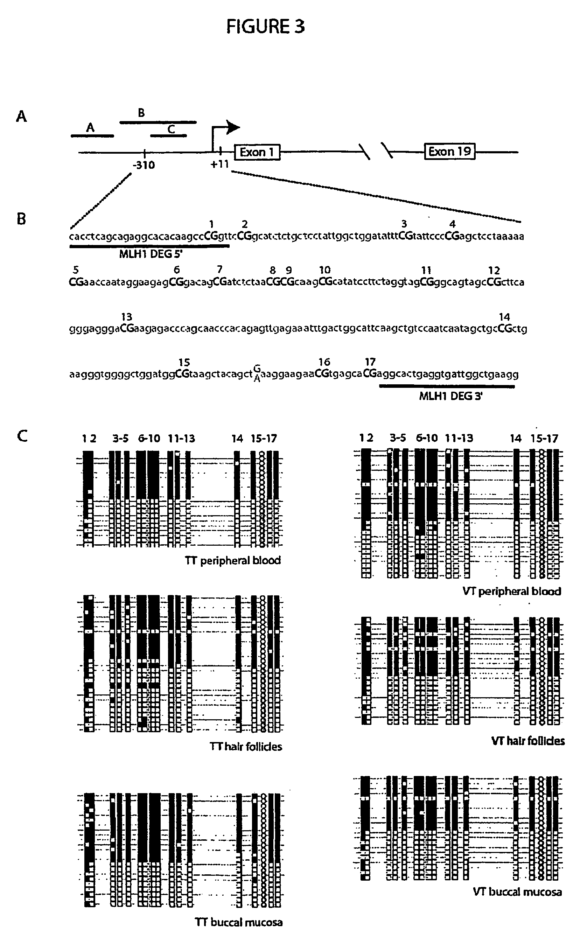

**Figure 3** shows bisulfite sequencing analysis of VT and TT somatic tissues in Example 1. (A) Schematic representation of the *hMLHT* locus showing the locations of the A, B, and C regions in relation to the region sequenced (dotted lines). (B) Sequence of *hMLH1* within the dotted region defined in (A). The primers used to amplify this region are underlined. CpG doublets within this domain are highlighted in bold and numbered 1 through 17. The single nucleotide polymorphism is also highlighted with G and A shown in larger text. (C) This figure shows the results of bisulfite allelic sequencing in the various somatic tissues of TT and VT. Black and white squares represent individual CpGs and are numbered according to their location in the sequence shown in (B). Grey or white circles represent the A or G genotype, respectively. Each horizontal row of squares represents the results from individual alleles. In both patient TT and VT, the hypermethylated alleles are always of the G genotype, whereas the A alleles show patchy methylation only and are never hypermethylated. Mosaicism was evident in the hair follicles of TT, and in all tissues from VT, as evidenced by the hypomethylation of the occasional G allele.

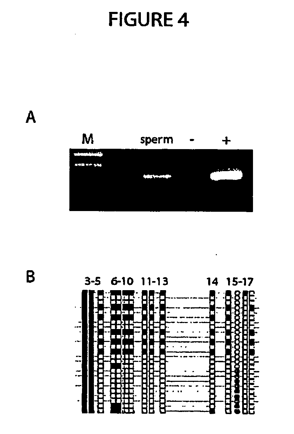

Figure 4 shows the results of bisulfite sequencing analysis of TT sperm in Example 1. (A) This photograph shows the results of the hybrid MSP-COBRA PCR used to amplify methylated alleles from the purified sperm from patient TT. Note the weak amplification of sperm compared to the positive control cell line (+). No amplification was seen in the negative control (peripheral blood from a healthy donor). (B) This figure shows the results of bisulfite allelic sequencing of the fragment from sperm shown in (A). Black and white squares represent individual CpGs and are numbered according to their location in the sequence shown in Figure 3(B). Grey or white circles represent the A or G genotype, respectively. Each horizontal row of squares represents the results from individual alleles. In the sperm, only G alleles were hypermethylated whereas the A alleles are hypomethylated. Mosaicism was evident with 10 of 16 G alleles demonstrating hypomethylation.

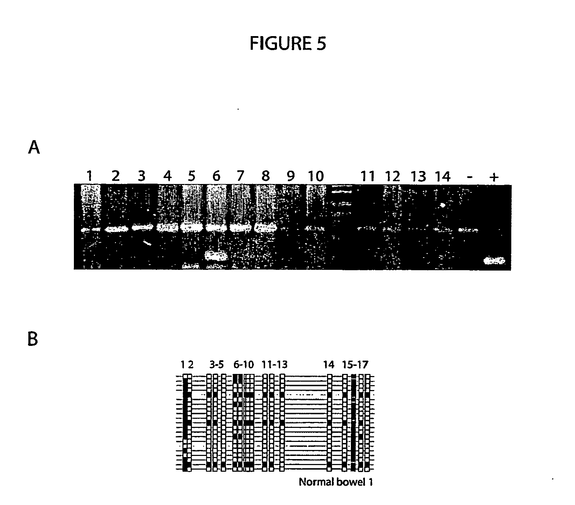

**Figure 5** provides the results of analysis of *hMLH1* methylation in normal bowel tissue from cancer patients in Example 1. (A) This photograph shows an example of the COBRA screening assay in the normal bowel tissue from 14 cancer patients. Shown are the results from the *hMLH1* C region COBRA. In this subset, one patient showed methylation of *hMLH1* in the C region in normal bowel tissue, demonstrated by digestion of the PCR product (upper band) to yield two smaller fragments, which appear as one band (arrow). (B) This figure shows the results of bisulfite allelic sequencing of the fragment from the normal bowel shown in (A). Black and white squares represent individual CpGs and are numbered according to their location in the sequence shown in Figure 3B. White circles represent the G genotype. Each horizontal row of squares represents the results from individual alleles. Hypermethylated alleles were clearly present and were always of the G genotype. This patient is a GG homozygote for the *hMLH1* SNP thus mosaicism cannot be determined.

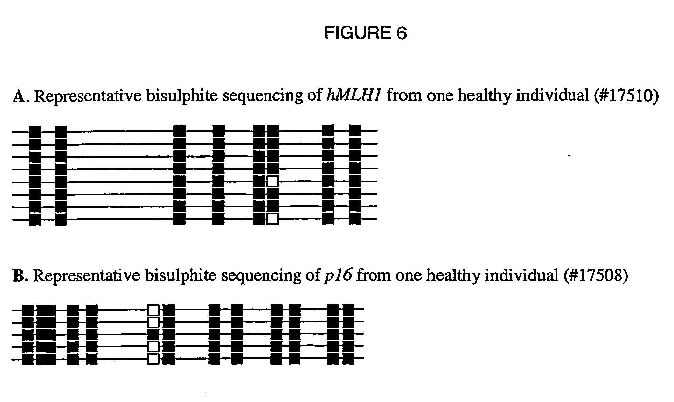

**Figure 6** provides representative bisulfite sequencing of MSP products from healthy individuals assayed in Example 2. Each horizontal row of squares represents the results from individual alleles. Black and white squares represent individual CpGs that are either methylated, or unmethylated, respectively. Hypermethylated alleles were dearly present in healthy individuals in both the *hMLH1* (A) and *p16* (B) genes.

### DETAILED DESCRIPTION OF THE INVENTION

The present invention provides an assay as defined in the claims

The determined epimutation frequency in the population of cells is predictive of disease risk (ie predictive of a predisposition to said disease) in said individual. For example, a positive risk of disease (ie a predisposition to disease) may be predicted by a determined epimutation frequency of at least 1 in 1x10⁶ cells or, more preferably, at least 1 in 1x10³ cells or, most preferably, at least 1 in 5x10² cells. Predictive frequencies of the epimutation may vary according to the source of the cells assayed. That is, the cells used in the assay may be from normal tissues such as, for example, normal peripheral blood, normal hair follicles and normal tissue from the buccal cavity, and determined frequencies which are predictive of disease risk may vary across those different normal tissue types.

As used herein, the term "normal tissue" refers to any tissue which is substantially healthy and not showing any significant symptoms or signs of disease (eg the tissue is not cancerous) and includes all normal somatic tissues. As indicated above, the cells used in the assay may be from normal peripheral blood, normal hair follicles and normal tissue from the buccal cavity. In addition to these, cells suitable for assaying may be from other normal somatic tissues including normal colonic mucosa. Preferably, the cells used in the assay are from normal peripheral blood.

The assayed epimutation is DNA methylation

The assayed methylation is one which is associated with the cancer for which a predisposition is to be assessed. For example, the epimutation is present in a chromosomal locus comprising a gene implicated in the manifestation or development of a disease.

The assayed epimutation may be present in the promoter of the gene(s) or other regulatory region of the gene(s) and is associated with transcriptional silencing of the gene(s).

The assayed methylation is present in a tumour suppressor gene such as *hMLH1, hMSH2, APC1A, APC1B* and *p16.*

Most preferably, the assayed epimutation is present in *hMLH1.*

The determination of the epimutation frequency in the assayed population of cells may be achieved by either directly assaying methylated cytosine (or other modification) on individual DNA strands or by otherwise assaying pooled DNA/chromatin, without examining individual DNA strands.

In order that the nature of the present invention may be more clearly understood, preferred forms thereof will now be described with reference to the following nonlimiting example.

### EXAMPLE 1

### Materials and Methods

### Patient samples

188 individuals with a personal history of cancer from St Vincent's Hospital (Sydney, NSW, Australia) and a further 50 individuals from the Victorian Clinical Genetics Service (Melbourne, VIC, Australia) were included in this study. Of these individuals, 65 were mutation-negative following screening for deleterious germ-line changes in *hMSH2, hMLH1* or *APC*, while 18 had hyperplastic polyposis and the remaining 155 only had a personal history of colorectal cancer.

DNA was extracted from peripheral blood, histologically-normal colonic mucosa, buccal smears, hair follicles and sperm using a standard phenol chloroform procedure (Sambrook et al 1989). To exclude the possibility of contaminating somatic cells in the sperm, semen was sorted on a FACSVantage DiVa (Becton Dickinson, Lexington, KY, USA) prior to DNA extraction. Sperm were identified on the basis of DNA content after propidium iodide staining as described (Schoell et al 1999). Purity of the sorted sperm was verified by FACS and microscopy.

### MSI analysis

Prior to the extraction of DNA from paraffin-embedded tissues, an adjacent section was examined histologically to ensure that it contained more than 60% tumour tissue. If this was not the case, foci of tumour were microdissected. The microsatellite status of each tumour was determined as previously described using the following primer sets: Bat 25, Bat 26, Bat 40, D5S346, D2S123, and D17S250 (Ward et al 2001). Tumours with instability at two or more markers were considered microsatellite unstable, while all others were designated as microsatellite stable (MSS).

### Immunohistochemical staining for hMSH2 and hMLH1

Immunohistochemical analysis was performed in a DAKO autostainer on dewaxed 4µm paraffin sections (DAKO Corporation, Carpinteria, CA, USA). Staining for hMLH1 and hMSH2 was as previously described, using monoclonal anti-human hMLH1 antibody (1:200, Becton Dickinson, Lexington, KY, USA) and monoclonal anti-human hMSH2 antibody (1:400, Pharmingen, San Diego, CA, USA). Expression of hMLH1 or hMSH2 was considered to be absent where there was no staining of tumour cells in the presence of nuclear staining in nearby germinal follicle lymphocytes or in epithelial cells in the base of adjacent non-neoplastic crypts. The immunostaining analysis was reported without knowledge of results of MSI, germ-line or CpG methylation results.

### Methylation screening assays

Genomic DNA (2µg) from each sample was then subject to bisulfite modification (Frommer et al 1992). COBRA (combined bisulfite and restriction analysis; Xiong and Laird 1997) was used as the screening test for all genes. DNA was screened for epimutations in a panel of 10 candidate gene promoters, namely *CDKN2A*, *hMLH1*, *HPP1*, *HIC1*, *RASSF1A*, *BRCA1*, *APC1A* and *1B*, *Blm* and *O⁶MGMT.* PCR primers, reaction conditions and post-PCR analysis information for the relevant assays are shown in Table 2. A maximum of 100 ng of bisulfite treated DNA was used in each reaction. In each PCR, positive and negative controls were included, and these were the cell line RKO (gift from M Brattain) and the peripheral blood DNA of a healthy donor, respectively.

### Allelotyping of the hMLH1 promoter

The single nucleotide polymorphism (SNP) at position -33 relative to the *hMLH1* transcription start site was determined by RFLP analysis of unmodified genomic DNA. Primers, reaction conditions and restriction digestion details are shown in Table 2.

### Bisulfite allelic sequencing

Samples demonstrating a positive result on the COBRA screening assay were examined in more detail using bisulfite sequencing. For *hMLH1,* this involved a degenerate PCR designed to amplify both unmethylated and methylated alleles, followed by cloning (pGEM-T, Promega) and sequencing of individual alleles with the BigDyes system (ABI). The primers and PCR conditions for amplification of this fragment are shown in Table 2. This fragment was designed to include the G/A SNP described above.

Bisulfite-modified sperm DNA was analysed with a hybrid PCR strategy, using a methylation-specific 5' primer and a methylation-degenerate 3' primer, to enrich for methylated sequences (Table 2).

### Results and Discussion

### Methylation of hMLH1 is present in all adult tissue from two individuals

Methylation at *hMLH1* was detected in DNA from peripheral blood cells of two of 94 individuals screened (Fig 1A). The methylation extended across the entire *hMLH1* promoter, which is encompassed by regions named A, B and C (Fig 1B). Similar results were found when the A, B and C screening assays were applied to DNA derived from these patients' hair follicles, and buccal mucosa. These unrelated individuals, TT (male) and VT (female) were aged 64 and 65 years, and both had a personal history of multiple primary malignancies which had been successfully treated with surgery alone. Clinical details of these individuals, as well as the results of immunostaining for the mismatch repair proteins and microsatellite testing of their tumours are shown in Table 3. All tumours tested exhibited microsatellite instability (MSI), which is a hallmark of mismatch repair deficiency. Loss of hMLH1 protein was confirmed in each tumour by immunohistochemistry in the presence of normal staining for hMSH2 (Fig 2). While TT and VT had previously undergone germ-line testing for mutations in the mismatch repair genes, no deleterious mutations had been identified despite extensive screening with a number of different methodologies.

To determine the distribution of CpG methylation within the tissues of TT and VT, and construct detailed methylation maps, bisulfite allelic sequencing was performed. By RFLP analysis, both TT and VT were found to be heterozygous for a G/A single nucleotide polymorphism in *hMLH1* at position -33 relative to the transcription start site. Bisulfite sequencing confirmed heterozygosity and also revealed that the methylation in all tissues tested was restricted to the one parental allele (Fig 3). In both individuals, the affected allele was the G genotype. The A allele was never found to be hypermethylated in any tissue, although some CpG sites were occasionally methylated on some alleles. The significance of this patchy methylation is unknown, but it is not likely to be associated with *hMLH1* silencing.

Mosaic methylation was observed in certain tissues from both patients (Fig 3). Patient VT was found to harbour some hypomethylated G alleles in her peripheral blood (1/15), hair follicles (2/12) and buccal mucosa (1/11). Of somatic tissues, patient TT exhibited mosaicism in hair follicles only, with 1 out of 15 G alleles being hypomethylated. Mosaicism was most evident in the sperm from TT. While the COBRA assay yielded variably weak and negative results (data not shown), methylated alleles were dearly present when the sperm were tested using an MSP-COBRA hybrid PCR (Fig 4). Of the alleles amplified, only G alleles (6/16) showed hypermethylation. This is not an indication of frequency however, because of the biased nature of the hybrid PCR. However, based on the limit of sensitivity of the assays, it is estimated that between 1 in 500 - 1 in 1000 alleles may be methylated in the sperm of patient TT.

### Methylation of hMLH1 in colonic mucosa of individuals with cancer

Normal colon tissue (n=133) from individuals with colorectal cancer arising either sporadically or in the setting of hyperplastic polyposis, was also screened. One individual with CpG methylation of *hMLH1* in the normal colon tissue was identified (Fig 5A). This individual (NB1, a 65 yr old male), was assessed in more detail with bisulfite sequencing (Fig 5B), and was found to be homozygous for the G SNP at -33 in *hMLH1,* thus rendering the determination of the level of mosaicism impossible. However, sequencing did confirm that NB1 harboured a significant percentage of hypermethylated *hMLH1* alleles in his normal colon (ie 14%; 3/21 alleles). It was also possible to assess the peripheral blood, hair and buccal mucosa of NB1 for *hMLH1* methylation by COBRA, although all assays were negative. Patient NB1 had developed a renal cell cancer at age 57 years and synchronous colorectal cancers in the caecum (microsatellite unstable) and sigmoid colon (microsatellite stable) at the age of 63 years in the setting of hyperplastic polyposis.

### Methylation of othergenes

All tested patients were negative for methylation at *CDKM24, hMLH2, HPP1, HIC1, RASSF1A, BRCA1, APC1A* and *1B, Blm* and *O⁶ MGMT.*

### EXAMPLE 2

### Methods and Materials

### Blood samples

Peripheral blood was collected from 22 healthy blood donors.

DNA was extracted from the peripheral blood as described in Example 1.

### Methylation screening assays

Genomic DNA (2 µg) from each peripheral blood sample was subjected to bisulfite modification as described in Example 1. The bisulfite-treated DNA was then subjected to PCR with methylation-specific primers (ie primers that hybridise with DNA in which the CpGs within the primer binding sites are methylated) for the *hMLH1 locus* and the *p16*locus (a tumour suppressor gene). Details of the primers used are listed in Table 4.

### Results and Discussion

Of the 22 healthy blood donors, 12 had some detectable level of hypermethylated *hMLH1* as demonstrated by the presence of a specific product following PCR with methylation-specificc primers. For the p16 gene, 18 of 29 healthy blood donors showed a detectable level of hypermethylated alleles. For both genes, the PCR products were confirmed as being hypermethylated by bisulfite sequencing. Representative sequencing is shown in Figure 6.

The results of this example indicate that healthy individuals commonly carry a detectable level of cells in which the *hMLH1* or *p16*gene is epimutated. Inactivation of one allele of either *hMLH1* or *p16,* and indeed many other tumour suppressor genes, is known to predispose a cell to become malignant through loss or inactivation of the second allele. In Example 1, two cancer patients (ie VT and TT) were described who carry an epimutation in all or nearly all of their somatic cells; the normal individuals studied in this example therefore presumably carry the epimutation in only a small proportion of their somatic cells. It is, however, considered that these cells are at risk of becoming malignant through loss or inactivation of the second allele, and this risk is higher than that of cells that do not carry the epimutation. Thus, it follows that the more cells in an individual that carry the epimutation, the higher will be that individual's risk of developing cancer; which is analogous to disease caused by mosaic carriage of a genetic mutation. Thus, the risk of developing cancer may be assessed by measuring the proportion of somatic cells carrying a particular epimutation. Also, the risk of developing other diseases that result from germline epimutation (particularly, if the disease results when only one allele is inactivated, ie haploin sufficiency, or when only a proportion of somatic cells are affected by this loss, ie mosaicism) ought similarly be assessed by measuring the proportion of somatic cells carrying a particular epimutation.

**Table 1 List of human diseases and genes associated with these disease phenotypes**

| **System Category** | **Disease** | **Genes Implicated (reference from Online Mendelian Inheritance in Man-OMIM)** |
|---|---|---|
| Blood Disease | Gaucher Disease | acid-beta glucosidase; HEMA (Factor VIII); Factor IX; Sphingomyelin phosphodiesterase-1; PIG-A; SLC11A3; TFR2; HLA-H |
| | Hemophilia A | |
| | Hemophilia B | |
| | NiemannPick Disease | |
| | Paroxysmal Nocturnal | |
| | Hemoglobinuria | |
| | Haemochromatosis von Willebrand disease | |
| Cancers | Including breast and ovarian cancer, malignant melanoma, multiple endocrine neoplasia, neurofibramatosis, pancreatic cancer, polycystic kidney disease, prostate cancer, retinoblastoma, leukaemia, lymphoma, tuberous sclerosis, von Hippel-Lindau syndrome, gastric cancer, renal cancer, endometrial cancer, paraganglioma, phaeochromocytoma; basal cell carcinoma; soft tissue sarcoma; brain tumours; testicular cancers; gynecological malignancies; | BRCA1; BRCA2; BRCA3; APC; hMLH1; hMSH2; hMSH6; hPMS1; hPMS2; CDKN2; MEN1; NF1; NF2; DPC4; PKD1; HPC1 locus; Rb; E cadherin; hamartin; tuberin; VHL; PRKARIA; PTEN; MMAC1; TEP1; CDK4; MET; SDHB; SDHC; SDHD; BMPR1A; p53; RET; PTC; WT; LKB1 |
| Digestive System | Ulcerative colitis ; Crohn's Disease Juvenile Onset Diabetes Wilson's Disease | CD19; sialophorin; CD11; IL-4 receptor IDDM1; IDDM2; IDDM3,-4, -5, -6, -7, -10, -12, -13,15, -18; GCK; INSR; AVPR2; ATP7B |
| Eye, Ear, Nose, and Throat | Deafness; Pendred Syndrome; Best Disease; Glaucoma; Gyrate Atrophy of the Choroid and Retina | connexion 26; pendrin; VMD2; GLC1A ornithine ketoacid aminotransferase |
| Endocrine | Adrenoleukodystrophy | ABCD1 |
| | Autoimmune Polyglandular | AIRE |
| | Syndrome | CSA; CSB |
| | Cockayne Syndrome | HNF-4α; glucokinase; HNF-1α; IPF-1; HNF-1β; |
| | Type II diabetes | NUEROD1; IRF-1; IPF1 |
| Cardiovascular | Ataxia Telangiectasia | ATM KCNQ1 dystrophin, lamin A/C; cardiac actin βmyosin heavy chain; α tropomyosin; cardiac troponin T and I; myosin binding protein C; myosin light chain; calcineurin; ABC1; angiotensinogen; β2 adrenergic receptor; alpha adducin; angiotensin-converting enzyme; serum paraoxonase; endothelial nitric oxide synthase; PAI-1; fibrinogen B; MTHFR; mineralocorticoid receptor ApoE; LDL receptor; hepatic lipase; |
| | Long QT Syndrome | |
| | Dilated cardiomyopathy | |
| | Hypertrophic cardiomyopathy | |
| | Tangier Disease | |
| | Essential hypertension | |
| | Eclampsia | |
| | Atherosclerosis | |
| | | |
| Immune System | DiGeorge Syndrome | DGS TNFSF5 6p21.3 (locus) IL2RG; JAK3; ADA MHC Class II genes CTLA-4; IL-2; AIRE |
| | Immunodeficiency with Hyper-IgM | |
| | IgA deficiency | |
| | Severe Combined | |
| | Immunodeficiency | |
| | Autoimmune diseases including | |
| | SLE, Sjogren syndrome, scleroderma, Type I diabetes, coeliac disease, inflammatory bowel disease, autoimmune haemolytic anaemia | |
| Muscle and Bone | Amyotrophic Lateral Sclerosis | SOD1 |
| | CharcotMarieTooth Syndrome | CMT loci |
| | Duchenne Muscular Dystrophy | dystrophin |
| | Marfan Syndrome | FBN1 |
| | Osteoarthritis | |
| Nervous System | Alzheimer Disease | PS1; PS2 |
| | Amyotrophic Lateral Sclerosis | SOD1; CNTF |
| | Angelman Syndrome | UBE3A |
| | Epilepsy | EPM2A |
| | Parkinson Disease | alpha-synuclein; UCHL1 |
| | Phenylketonuria | parkin |
| | Prader-Willi Syndrome | PAH |
| | Refsum Disease | SNRPN |
| | Rett Syndrome | PAHX |
| | Schizophrenia | MeCP2 |
| | Spinal Muscular Atrophy | Neuregulin; reelin |
| | Tay-Sachs Disease | SMN1 |
| | | HEXA |

**Table 2 Methods for amplification of various hMLH1 loci**

| ASSAY TYPE | LOCUS | PRIMERS AND SEQUENCE 5'-3' | PCR CYCLING* | PRODUCT SIZE | POST-PCR ANALYSIS |
|---|---|---|---|---|---|
| COBRA | MLH1-A -691 to -442 | MLH1 AF | 1x 94°C 5min | | Digest product with 2U *TaiI.* If methylated, product will digest to yield 108 and 48 bp fragments |
| | | | 10x 94°C/30 sec; 65°C/60 sec (-1°/cycle) | 155 bp | |
| | | MLH1 | 25x 94°C/30 sec; 55°C/45 sec | | |
| | MLH1-B -444 to-103 | MLH1 BF AAATTTTTTAATTTTGTGGGTTG (SEQ ID NO: 3) | 1x 94°C 5min | | Digest product with 2U *HinA.* If methylated, product will digest to yield 178 and 164 bp fragments |
| | | MLH1 BAR | 10x 94°C/30 sec; 60°C/60 sec (-1°/cycle) | 344 bp | |
| | | | 25x 94°C/30 sec; 50°C/45 sec | | |
| | MLH1-C -291 to -42 | MLH1 CF | 1x 94°C 5min | | Digest product with 2U *BstU1.* If methylated, product will digest to yield 74 and 69 bp fragments |
| | | | 10x 94°C/30 sec; 65°C/60 sec (-1°/cycle) | 140 bp | |
| | | MLH1 CR | 25x 94°C/30 sec; 55°C/45 sec | | |
| ALLELOTY PING | G/A SNP at-33 | MLH1 SNP5 | 1x 94°C 5min | | Digest product with *Pvu*II A/A genotype will not digest A/G will partially digest to yield 296, 247 and 40bp fragments G/G will digest completely to 247 and 40bp |
| | | | 10x 94°C/30 sec; 65°C/60 sec (-1°/cycle) | 296 bp | |
| | | MLH1SNP3 | 20x 94°C/30 sec; 55°C/45 sec | | |
| ALLELIC SEQUENCI NG | MLH1 -372 to -52 | MLH1 DEG 5 TATTTTAGTAGAGGTATATAAGTTYGG (SEQ ID NO: 9) | 1x 94°C 5min | | Product cloned into P-GEM-T vector and individual clones are sequenced |
| | | | 10x 94°C/30 sec; 62°C/60 sec (-1°/cycle) | 324 bp | |
| | | MLH1 DEG 3CCTTCAACCAATCACCTCAATACC (SEQ ID NO: 10) | 26x 94°C/30 sec; 52°C/45 sec | | |
| HYBRID PCR | MLH1 -326 to -52 | MLH1 MSP 5 ATTGGTTGGATATTTCGTATTTITC ((SEQ ID NO: 11) | 1x 94°C 5min | | Product cloned into P-GEM-T vector and individual clones are sequenced |
| | | MLH1 DEG 3 CCTTCAACCAATCACCTCAATACC(SEQ ID NO: 12) | 10x 94°C/30 sec; 62°C/60 sec(-1°/cycle) | 276 bp | |
| | | | 27x 94°C/30 sec; 52°C/45 sec | | |

| | | | | | |
|---|---|---|---|---|---|
| *All reactions performed with FastStart Taq polymerase with 1 uM each primer, 2mM MgCl₂ and 0.25 mM dNTPs | | | | | |

**Table 3 Results of microsatellite testing and immunostaining on tumours from two individuals (VT and TT) with hMLH1 methylation of Example 1. The age at which each tumour developed is shown. H = microsatellite instability, ND is not done because of insufficient DNA or tumour was unavailable. Immunostaining is shown as present (Pos), absent (Neg) or not done (ND).**

| | **Cancer** | **Age** | **MLH1 IHC** | **MSH2 IHC** | **MSI** |
|---|---|---|---|---|---|
| TT | Colorectal - caecum | 43 | Neg | Pos | H |
| | Colorectal - descending colon | 44 | ND | ND | ND |
| | Duodenal - 3 synchronous tumours | 51 | Neg | Pos | H |
| | Ampulla of Vater | 59 | Neg | Pos | H |
| VT | Colorectal - caecum | 46 | Neg | Pos | H |
| | Endometrial | 53 | Neg | Pos | H |
| | Melanoma | 57 | ND | ND | ND |
| | Breast - infiltrating ductal | 63 | Neg | Pos | H |

**Table 4 Primers used in methylation-specific PCR in Example 2**

| Locus | Primer sequence 5'-3' | PCR cycling | Product size |
|---|---|---|---|
| | forward: | 1x 94°C 5min | 202 |
| ***hMLH1*** | | 10x 94°C/30 sec; 62°C/60 sec(-1°/cycle) | |
| -162 to +40 | reverse: | | |
| | | 30x 94°C/30sec;52°C/45sec | |
| | forward: | 1x 94°C 5min | 96 |
| ***p16*** | | 10x 94°C/30 sec; 65°C/60 sec(-1°/cycle) | |
| +256 to +352 | reverse: | | |
| | | 30x 94°C/30 sec; 55°C/45 sec | |

### REFERENCES

1. Antequera, F. and A. Bird.1993. CpG islands. Exs 64: 169-85*.*
2. Baylin, S.B. and J.G. Herman. 2000. DNA hypermethylation in tumorigenesis: epigenetics joins genetics. Trends Genet 16: 168-74.
3. Bird, A. 2002. DNA methylation patterns and epigenetic memory. Genes Dev 16: 6-21.
4. Botstein, D. and N. Risch. 2003. Discovering genotypes underlying human phenotypes: past successes for mendelian disease, future approaches for complex disease. Nat Genet 33: 228-37.
5. Cubas, P., C. Vincent, and E. Coen. 1999. An epigenetic mutation responsible for natural variation in floral symmetry. Nature 401: 157-61.
6. Cui, H., M. Cruz-Correa, F.M. Giardiello, D.F. Hutcheon, D.R. Kafonek, S. Brandenburg, Y. Wu, X. He, N.R. Powe, and A.P. Feinberg. 2003. Loss of IGF2 imprinting: a potential marker of colorectal cancer risk. Science 299: 1753-5.
7. Cui, H., I.L. Horon, R. Ohlsson, S.R. Hamilton, and A.P. Feinberg. 1998. Loss of imprinting in normal tissue of colorectal cancer patients with microsatellite instability. Nat Med 4: 1276-80.
8. Cui, H., P. Onyango, S. Brandenburg, Y. Wu, C.L. Hsieh, and A.P. Feinberg. 2002. Loss of imprinting in colorectal cancer linked to hypomethylation of H19 and IGF2. Cancer Res 62: 6442-6.
9. Duhl, D.M., H. Vrieling, K.A. Miller, G.L. Wolff, and G.S. Barsh. 1994. Geomorphic agouti mutations in obese yellow mice. Nat Genet 8: 59-65.
10. Frommer, M., L.E. McDonald, D.S. Millar, C.M. Collis, F. Watt, G.W. Grigg, P.L. Molloy, and C.L. Paul. 1992. A genomic sequencing protocol that yields a positive display of 5-methylcytosine residues in individual DNA strands. Proc Natl Acad Sci USA 89:1827-31.
11. Herman, J.G., A. Umar, K. Polyak, J.R. Graff, N. Ahuja, J.P. Issa, S. Markowitz, J.K. Willson, S.R. Hamilton, K.W. Kinzler, M.F. Kane, R.D. Kolodner, B. Vogelstein, T.A. Kunkel, and S.B. Baylin. 1998. Incidence and functional consequences of hMLH1 promoter hypermethylation in colorectal carcinoma. Proc Natl Acad Sci USA 95: 6870-5.
12. Holliday, R. 1987. The inheritance of epigenetic defects. Science 238: 163-70.
13. Jenuwein, T. and C.D. Allis. 2001. Translating the histone code. Science 293: 1074-80.
14. Jones, P.A. and P.W. Laird. 1999. Cancer epigenetics comes of age. Nat Genet 21: 163-7.
15. Jones, P.A. and D. Takai. 2001. The role of DNA methylation in mammalian epigenetics. Science 293:1068-70*.*
16. Kouzarides, T. 2002. Histone methylation in transcriptional control. Curr Opin Genet Dev 12:198-209.
17. Morgan, H.D., H.G. Sutherland, D.I. Martin, and E. Whitelaw. 1999. Epigenetic inheritance at the agouti locus in the mouse. Nat Genet 23: 314-8.
18. Risch, N.J. 2000. Searching for genetic determinants in the new millennium. Nature 405: 847-56.
19. Sambrook, J., E.F. Fritsch, and T. Maniatis. 1989. Molecular cloning. A laboratory manual. Cold Spring Harbor Laboratory Press, Cold Spring Harbor.
20. Schoell, W.M., M. Klintschar, R. Mirhashemi, and B. Pertl. 1999. Separation of sperm and vaginal cells with flow cytometry for DNA typing after sexual assault. Obstet Gynecol 94: 623-7.
21. Tamaru, H. and E.U. Selker. 2001. A histone H3 methyltransferase controls DNA methylation in Neurospora crassa. Nature 414: 277-83.
22. Ward, R., A. Meagher, I. Tomlinson, T. O'Connor, M. Norrie, R. Wu, and N. Hawkins. 2001. Microsatellite instability and the clinicopathological features of sporadic colorectal cancer. Gut48: 821-9*.*
23. Wheeler, J.M., N.E. Beck, H.C. Kim, I.P. Tomlinson, N.J. Mortensen, and W.F. Bodmer. 1999. Mechanisms of inactivation of mismatch repair genes in human colorectal cancer cell lines: the predominant role of hMLH1. Proc Natl Acad Sci USA 96:10296-301.
24. Wolffe, A.P. and M.A. Matzke. 1999. Epigenetics: regulation through repression. Science 286: 481-6.
25. Xiong, Z. and P.W. Laird. 1997. COBRA: a sensitive and quantitative DNA methylation assay. Nucleic Acids Res 25: 2532-4.

## Claims

1. An assay for assessing a predisposition to a cancer in a healthy individual, wherein said assay comprises
(A) measuring what proportion of non-malignant somatic cells isolated from a tissue of said individual carries an epimutation present in a tumour suppressor gene, the silencing of which is associated with cancer, and the epimutation being DNA methylation, wherein said tissue is substantially healthy and does not show any significant symptoms or signs of disease, and
(B) assessing said predisposition based on said proportion, wherein said gene is other than one that is subject to normal parent of origin-specific expression.

2. The assay of claim 1, wherein said somatic cells are from a normal tissue that is selected from the group consisting of normal peripheral blood, normal hair follicle tissue and normal tissue from the buccal cavity.

3. The assay of claim 1, wherein the normal tissue is normal peripheral blood.

4. The assay of any one of claims 1 to 3, wherein the epimutation is present in the promoter or other regulatory region of the gene and is associated with transcriptional silencing of said gene.

5. The assay of claim 1, wherein the epimutation is present in a gene selected from the group consisting of hMLH1, hMSH2, APC1A, APC1B and p16.

6. The assay of claim 5, wherein the epimutation is present in hMLH1.

7. The assay of claim 1, wherein said assessment indicates a predisposition to cancer when the epimutation frequency is at least 1 in 1 × 10⁶ cells.

8. The assay of claim 1, wherein said assessment indicates a predisposition to cancer when the epimutation frequency is at least 1 in 1 × 10³ cells.

9. The assay of claim 1, wherein said assessment indicates a predisposition to cancer when the epimutation frequency is at least 1 in 5 × 10² cells.

10. An assay for assessing a predisposition to a cancer in a tissue in a healthy individual, wherein said assay comprises
(A) measuring what proportion of non-malignant somatic cells isolated from a different tissue from said individual carries an epimutation present in a tumour suppressor gene, the silencing of which is associated with cancer, wherein (i) the epimutation is DNA methylation and (ii) said different tissue is substantially healthy and does not show any significant symptoms or signs of disease, and
(B) assessing said predisposition based on said proportion, wherein said gene is other than one that is subject to normal parent of origin-specific expression, and wherein the different tissue is a normal tissue selected from the group consisting of normal peripheral blood, normal hair follicle tissue, and normal tissue from the buccal cavity.

## Patentansprüche

1. Assay zur Beurteilung einer Prädisposition für einen Krebs bei einer gesunden Person, wobei das Assay umfasst
(A) Messen des Anteils an nicht malignen somatischen Zellen, die von einer Gewebeprobe der Person isoliert wurden, mit einer Epimutation in einem Tumorsuppressorgen, dessen Stilllegung mit Krebs assoziiert ist, wobei die Epimutation eine DNA-Methylierung ist, wobei das Gewebe im Wesentlichen gesund ist und keine wesentlichen Krankheitssymptome oder -zeichen zeigt, und
(B) Beurteilen der Prädisposition auf der Grundlage des Anteils, wobei das Gen von einem Gen verschieden ist, das einer normalen parent-of-originspezifischen Expression unterworfen wird.

2. Assay nach Anspruch 1, wobei die somatischen Zellen von einem normalen Gewebe stammen, ausgewählt aus der Gruppe, bestehend aus normalem pheripherem Blut, normalem Haarfollikelgewebe und normalem Gewebe der Mundhöhle.

3. Assay nach Anspruch 1, wobei das normale Gewebe normales peripheres Blut ist.

4. Assay nach einem der Ansprüche 1 bis 3, wobei die Epimutation in dem Promotor oder einer anderen Regulierungsregion des Gens auftritt und mit einer transkriptionellen Stilllegung des Gens assoziiert ist.

5. Assay nach Anspruch 1, wobei die Epimutation in einem Gen auftritt, ausgewählt aus der Gruppe, bestehend aus hMLH1, hMSH2, APC1A, APC1B und p16.

6. Assay nach Anspruch 5, wobei die Epimutation in hMLH1 auftritt.

7. Assay nach Anspruch 1, wobei die Beurteilung eine Prädisposition für Krebs anzeigt, wenn die Epimutationshäufigkeit mindestens 1 in 1 × 10⁶ Zellen ist.

8. Assay nach Anspruch 1, wobei die Beurteilung eine Prädisposition für Krebs anzeigt, wenn die Epimutationshäufigkeit mindestens 1 in 1 × 10³ Zellen ist.

9. Assay nach Anspruch 1, wobei die Beurteilung eine Prädisposition für Krebs anzeigt, wenn die Epimutationshäufigkeit mindestens 1 in 5 × 10² Zellen ist.

10. Assay zur Beurteilung einer Prädisposition für einen Krebs in einem Gewebe einer gesunden Person, wobei das Assay umfasst
(A) Messen des Anteils an nicht malignen somatischen Zellen, die von einem anderen Gewebe der Person isoliert wurden, mit einer Epimutation in einem Tumorsuppressorgen, dessen Stilllegung mit Krebs assoziiert ist, wobei (i) die Epimutation eine DNA-Methylierung ist und (ii) das andere Gewebe im Wesentlichen gesund ist und keine wesentlichen Krankheitssymptome oder -zeichen zeigt, und
(B) Beurteilen der Prädisposition auf der Grundlage des Anteils, wobei das Gen von einem Gen verschieden ist, das einer normalen parentof-origin-spezifischen Expression unterworfen wird, und wobei das andere Gewebe ein normales Gewebe ist, ausgewählt aus der Gruppe, bestehend aus normalem pheripherem Blut, normalem Haarfollikelgewebe und normalem Gewebe der Mundhöhle.

## Revendications

1. Test destiné à évaluer une prédisposition à un cancer chez un individu sain, dans lequel ledit test comprend
(A) mesure de la proportion de cellules somatiques bénignes isolées à partir d'un tissu provenant dudit individu qui sont porteuses d'une modification épigénétique présente dans un gène suppresseur de tumeur, dont le silençage est associé au cancer, et la modification épigénétique étant une méthylation de l'ADN, dans laquelle ledit tissu est essentiellement sain et ne présente aucun symptôme ou signe significatif de maladie, et
(B) évaluation de ladite prédisposition sur la base de ladite proportion, dans laquelle ledit gène est autre qu'un gène sujet à une expression normale déterminée par le parent d'origine.

2. Le test de la revendication 1, dans lequel lesdites cellules somatiques proviennent d'un tissu normal qui est choisi dans le groupe consistant en sang périphérique normal, tissu de follicule pileux normal et tissu normal provenant de la cavité buccale.

3. Le test de la revendication 1, dans lequel le tissu normal est du sang périphérique normal.

4. Le test de l'une quelconque des revendications 1 à 3, dans lequel la modification épigénétique est présente dans le promoteur ou une autre région régulatrice du gène et est associée au silençage transcriptionnel dudit gène.

5. Le test de la revendication 1, dans lequel la modification épigénétique est présente dans un gène choisi dans le groupe consistant en hMLH1, hMSH2, APC1A, APC1B et p16.

6. Le test de la revendication 5, dans lequel la modification épigénétique est présente dans hMLH1.

7. Le test de la revendication 1, dans lequel ladite évaluation indique une prédisposition au cancer quand la fréquence de la modification épigénétique est au moins de 1 pour 1 × 10⁶ cellules.

8. Le test de la revendication 1, dans lequel ladite évaluation indique une prédisposition au cancer quand la fréquence de la modification épigénétique est au moins de 1 pour 1 × 10³ cellules.

9. Le test de la revendication 1, dans lequel ladite évaluation indique une prédisposition au cancer quand la fréquence de la modification épigénétique est au moins de 1 pour 5 × 10² cellules.

10. Test destiné à évaluer une prédisposition à un cancer dans un tissu chez un individu sain, dans lequel ledit test comprend
(A) mesure de la proportion de cellules somatiques bénignes isolées à partir d'un tissu différent provenant dudit individu qui sont porteuses d'une modification épigénétique présente dans un gène suppresseur de tumeur, dont le silençage est associé au cancer, dans laquelle (i) la modification épigénétique est une méthylation de l'ADN et (ii) ledit tissu différent est essentiellement sain et ne présente aucun symptôme ou signe significatif de maladie, et
(B) évaluation de ladite prédisposition sur la base de ladite proportion, dans laquelle ledit gène est autre qu'un gène sujet à une expression normale déterminée par le parent d'origine, et dans laquelle le tissu différent est un tissu normal choisi dans le groupe consistant en sang périphérique normal, tissu de follicule pileux normal et tissu normal provenant de la cavité buccale.


### SEQUENCE LISTING

<110> St Vincent's Hospital (Sydney) Limited and Victor Chang Cardiac Research Institute
<120> Assessment of disease risk by determining frequency of gene epimutation
   <130> 03 1373 9247
   <160> 16
   <170> PatentIn version 3.3
<210> 1
   <211> 26
   <212> DNA
   <213> Artificial
<220>
   <223> primer sequence MLH1 AF
<400> 1
   ttaygggtaa gtygttttga ygtaga
   26
<210> 2
   <211> 26
   <212> DNA
   <213> Artificial
<220>
   <223> primer sequence MLH1 AR
<400> 2
   cctataccta atctatcrcc rcctca
   26
<210> 3
   <211> 23
   <212> DNA
   <213> Artificial
<220>
   <223> primer sequence MLH1 BF
<400> 3
   aaatttttta attttgtggg ttg
   23
<210> 4
   <211> 24
   <212> DNA
   <213> Artificial
<220>
   <223> primer sequence MLH1 BR
<400> 4
   acttccatct tacttctttt aaac 24
<210> 5
   <211> 25
   <212> DNA
   <213> Artificial
<220>
   <223> primer sequence MLH1 CF
<400> 5
   ggttggatat ttygtatttt tygag 25
<210> 6
   <211> 25
   <212> DNA
   <213> Artificial
<220>
   <223> primer sequence MLH1 CR
<400> 6
   aattactaaa tctcttcrtc cctcc 25
<210> 7
   <211> 26
   <212> DNA
   <213> Artificial
<220>
   <223> primer sequence MLH1 SNP 5
<400> 7
   gcatctctgc tcctattggc tggata 26
<210> 8
   <211> 26
   <212> DNA
   <213> Artificial
<220>
   <223> primer sequence MLH1 SNP 3
<400> 8
   agtgccttca gccaatcacc tcagtg 26
<210> 9
   <211> 27
   <212> DNA
   <213> Artificial
<220>
   <223> primer sequence MLH1 DEG 5
<400> 9
   tattttagta gaggtatata agttygg
   27
<210> 10
   <211> 24
   <212> DNA
   <213> Artificial
<220>
   <223> primer sequence MLH1 DEG 3
<400> 10
   ccttcaacca atcacctcaa tacc
   24
<210> 11
   <211> 25
   <212> DNA
   <213> Artificial
<220>
   <223> primer sequence MLH1 MSP 5
<400> 11
   attggttgga tatttcgtat ttttc
   25
<210> 12
   <211> 24
   <212> DNA
   <213> Artificial
<220>
   <223> primer sequence MLH1 DEG 3
<400> 12
   ccttcaacca atcacctcaa tacc
   24
<210> 13
   <211> 23
   <212> DNA
   <213> Artificial
<220>
   <223> hMLH1 forward primer
<400> 13
   tagtagtcgt tttagggagg gac
   23
<210> 14
   <211> 25
   <212> DNA
   <213> Artificial
<220>
   <223> hMLH1 reverse primer
<400> 14
   aaaaaacgtc taaatactca acgaa
   25
<210> 15
   <211> 20
   <212> DNA
   <213> Artificial
<220>
   <223> p16 forward primer
<400> 15
   gttggttacg gtcgcggttc
   20
<210> 16
   <211> 22
   <212> DNA
   <213> Artificial
<220>
   <223> p16 reverse primer
<400> 16
   ccgaccgtaa ctattcgata cg
   22